# EUROPEAN PATENT APPLICATION

(11) **EP 3 516 983 A1**
(43) Date of publication of application: **31.07.2019**
(21) Application number: 17853033.3
(22) Date of filing: 19.09.2017
(51) Int. Cl.: A45D 34/04, A61M 35/00

(54) **DISPOSABLE APPLICATOR**

(30) Priority: 20.09.2016 JP 2016183227
(71) Applicant: Shiseido Company, Ltd., Tokyo 104-0061 (JP)
(72) Inventor: MATSUSHITA, Akane, Yokohama-shi, Kanagawa 224-8558 (JP); KUROKAWA, Tomohiro, Takayama-shi, Gifu 506-0041 (JP); SUGA, Emiko, Tokyo 110-8560 (JP)
(74) Representative: Ter Meer Steinmeister & Partner
(86) International application number: PCT/JP2017/033727
(87) International publication number: WO 2018/056261

(57) **Abstract**

The present invention provides an applicator used for applying viscous cosmetics such as lip gloss and viscous pharmaceutical preparations such as ointments, the applicator being sanitary and disposable but capable of reliably scooping the cosmetic or pharmaceutical preparation, coming into firm contact with the application area, and easily spreading the cosmetic or pharmaceutical preparation.

Provided is an applicator 1 having a cotton body 3 that extends from an end portion of a shaft 2 and has a flat curved shape in which a maximum width W is 4.5 to 6.5 mm and a thickness T at a thickest portion is 2.0 to 3.6 mm, wherein an imaginary line 4 connecting a tip portion of the cotton body and a rear end central portion thereof has an inclination angle of 25 to 35 degrees with respect to the shaft 2.

## Description

### [Technical Field]

The present invention relates to an applicator for applying viscous cosmetics such as lip gloss and viscous pharmaceutical preparations such as ointments.

### [Background Art]

Viscous cosmetics such as lip gloss have been popular among consumers due to the cosmetic effects thereof that give lips a glossy luster to create three-dimensional lip looks, and viscous cosmetics of a wide variety of colors and textures are sold in the market.

When a consumer purchases a viscous cosmetic at a store, the consumer may apply samples of the viscous cosmetic to his/her lips and select the product suiting him/her most. Applicators used for applying samples are preferably disposable for sanitary reasons so that the same applicators are not used by the next consumers. Furthermore, samples of viscous cosmetics distributed as free testers preferably accompany disposable applicators that can be used once or several times until using up the cosmetics.

Although disposable, in order to evaluate a product using such a disposable applicator, the applicator needs to be designed to allow a consumer to scoop a sufficient amount of a viscous cosmetic and easily spread the cosmetic over the application area to adequately see the features of the product such as the color and texture thereof.

For these reasons, as a disposable applicator that is used by a consumer at a store or the like to purchase a viscous cosmetic such as lip gloss, a disposable viscous cosmetic applicator that is not used by other consumers and is therefore sanitary and capable of reliably scooping the cosmetic, coming into firm contact with the application area and easily spreading the cosmetic needs to be developed. Even for viscous pharmaceutical preparations such as ointments, it is crucial to develop sanitary, disposable applicators that are used only once and capable of easily scooping and spreading the viscous preparations over the affected areas.

### [Citation List]

### [Patent Literature]

[PTL 1] Japanese Patent Application Laid-open No. 2003-199616

### [Summary of Invention]

### [Technical Problem]

An object of the present invention is to provide an applicator used for applying viscous cosmetics such as lip gloss and viscous pharmaceutical preparations such as ointments, the applicator being sanitary and disposable but capable of reliably scooping the cosmetic or pharmaceutical preparation, coming into firm contact with the application area, and easily spreading the cosmetic or pharmaceutical preparation.

### [Solution to Problem]

As a result of studies in order to achieve the foregoing object, the inventors of the present invention have discovered an applicator which, while sanitary and disposable, can reliably scoop a viscous cosmetic such as lip gloss, come into firm contact with the application area, and easily spread the viscous cosmetic, by shaping a cotton body extending from an end portion of a shaft into a predetermined flat curved shaped. As a result, the inventors have completed the present invention.

Specifically, the present invention is an applicator having a cotton body that extends from an end portion of a shaft and has a flat curved shape in which a maximum width is 4.5 to 6.5 mm and a thickness at a thickest portion is 2.0 to 3.6 mm, wherein an imaginary line connecting a tip portion of the cotton body and a rear end central portion thereof has an inclination angle of 25 to 35 degrees with respect to the shaft.

Furthermore, the present invention is an applicator in which the cotton body is formed of an aggregate of cotton fibers bonded together with 0.5 mg of polyvinyl alcohol resin.

Moreover, the present invention is a viscous cosmetic applicator having a cotton body that extends from an end portion of a shaft and has a flat curved shape in which a maximum width is 4.5 to 6.5 mm and a thickness at a thickest portion is 2.0 to 3.6 mm, wherein an imaginary line connecting a tip portion of the cotton body and a rear end central portion thereof has an inclination angle of 25 to 35 degrees with respect to the shaft.

Further, the present invention is a viscous cosmetic applicator in which the cotton body is formed of an aggregate of cotton fibers bonded together with 0.5 mg of polyvinyl alcohol resin.

In addition, the present invention is a viscous cosmetic applicator capable of applying a viscous cosmetic having a viscosity of 400 to 300000 mPa·s.

Also, the present invention is a method for using an applicator, the method including scooping and applying a viscous cosmetic or a viscous pharmaceutical preparation by using the foregoing applicator.

Furthermore, the present invention a method for using a viscous cosmetic applicator, the method including scooping and applying a viscous cosmetic by using the foregoing viscous cosmetic applicator.

### [Advantageous Effects of Invention]

According to the applicator of the present invention, an applicator used for applying various viscous substances such as viscous cosmetics and viscous pharmaceutical preparations can be made sanitary and disposable and configured to reliably scoop a viscous cosmetic such as lip gloss, come into firm contact with the application area, and easily spread the viscous cosmetic thereon. Moreover, according to the applicator of the present invention, although disposable, the same excellent sense of usability as that of a resin applicator that normally accompanies a cosmetic container and an applicator with a flocking-finished surface.

Additionally, since the viscous cosmetic applicator of the present invention has necessary durability, the user can use a sample of a viscous cosmetic distributed as a free tester, once or several times repeatedly until using up the cosmetic.

### [Brief Description of Drawings]

[Fig. 1]

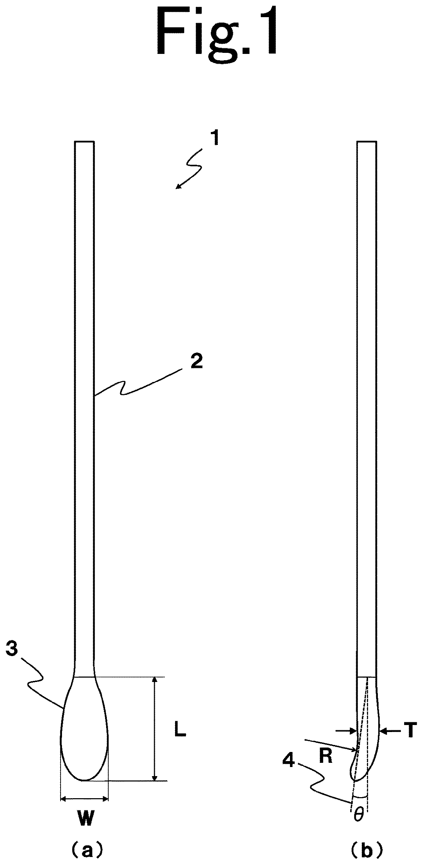

   Fig. 1 is an external view ((a) front view, (b) side view) of an applicator.
[Fig. 2]

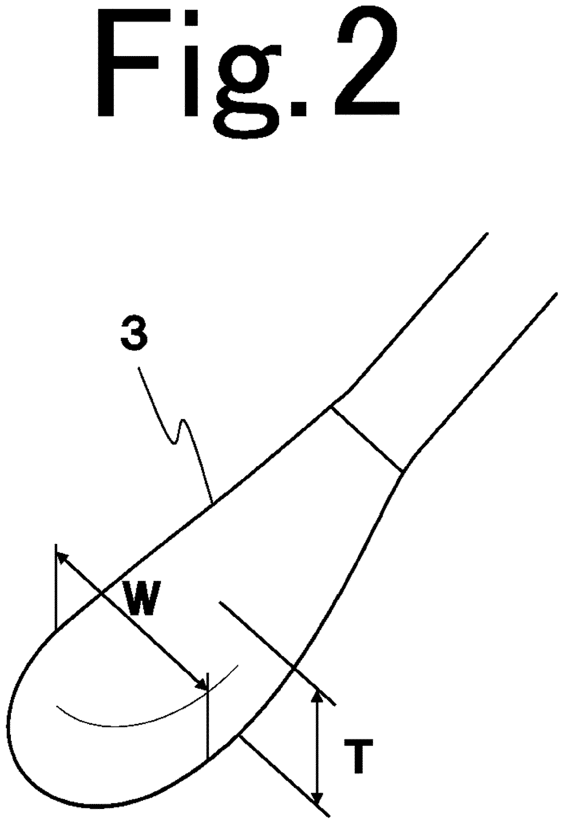

   Fig. 2 is an enlarged perspective view of a cotton body.
[Fig. 3]

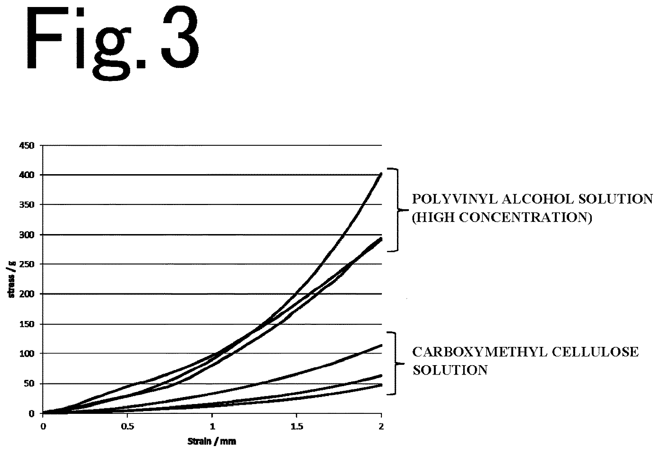

   Fig. 3 is a graph showing the hardness of the cotton body.
[Fig. 4]

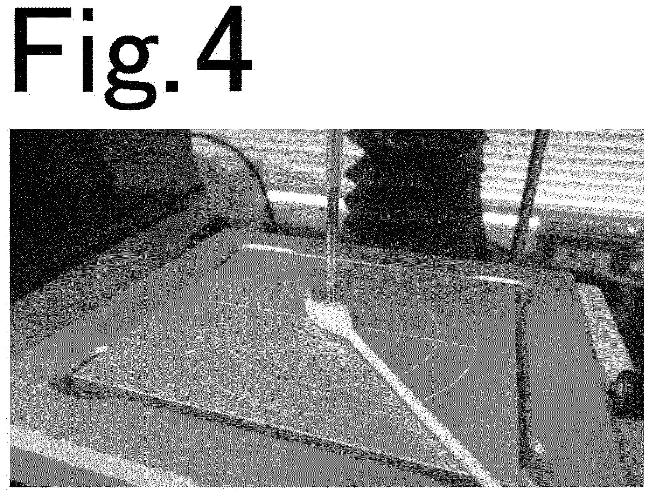

   Fig. 4 is a diagram showing how the hardness of the cotton body is measured (a diagram-substituted photograph).

### [Description of Embodiments]

As shown in Fig. 1, an applicator according to the present invention has a cotton body (3) extending from an end portion of a shaft (2), the cotton body having a flat curved shape in which a maximum width W is 4.5 to 6.5 mm and a thickness T at a thickest portion is 2.0 to 3.6 mm, and an imaginary line (4) connecting a tip portion of the cotton body and a rear end central portion thereof has an inclination angle θ of 25 to 35 degrees with respect to the shaft (2).

It is preferred that a viscous cosmetic normally have a viscosity of 4000 mPa·s or more in order to obtain a thick applied layer and bring about excellent gloss and luster (the measured value is obtained using BL-type viscometer manufactured by Shibaura Semtek Co., Ltd. under the measurement condition of rotor No. 4 at 0.3 rpm). It is particularly preferred that the viscosity of the viscous cosmetic be 10000 to 100000 mPa·s in order to achieve a balance between the ease of application and excellent luster.

The cotton body (3) has a flat curved shape so as to be able to reliably scoop the viscous cosmetic, come into firm contact with the application area, and easily spread the viscous cosmetic.

The maximum width W of the cotton body (3) is 4.5 to 6.5 mm. While the maximum width W smaller than 4.5 mm results in a smaller amount of the cosmetic to be scooped and hence an insufficient amount of the cosmetic to be applied, the maximum width W greater than 6.5 mm makes it difficult to apply the cosmetic to small, fine areas, making the applicator less user-friendly.

The thickness T of the thickest portion of the cotton body (3) is 2.0 to 3.6 mm. The thickness T of the thickest portion less than 2.0 mm makes the cotton body less rigid and consequently makes the cotton body become deformed easily, leading to difficulty in scooping and applying the viscos cosmetic. If the thickness T of the thickest portion is greater than 3.6 mm, however, the cotton body becomes excessively thick and scooping of the viscous cosmetic becomes difficult; the cosmetic cannot be applied to small, fine areas easily.

Also, the cotton body (3) has a shape that allows the imaginary line (4) to have the inclination angle θ of 25 to 35 with respect to the shaft, the imaginary line (4) connecting the tip portion of the cotton body and the rear end central portion thereof. Providing the inclination angle θ in the cotton body not only makes scooping of the cosmetic easy, but also allows the viscous cosmetic to be applied easily to the application area such as lips, enabling easy application of the viscous cosmetic and the like.

In addition, it is preferred that a length L of the cotton body (3) be 10 to 17 mm and that a curvature radius R of the curved surface be 4.5 to 5.2 mm. The reason is because, in a case where the viscos cosmetic is a lip gloss, the cotton body needs to have a shape allowing the cosmetic to easily stick to the application area, i.e., the lips.

A method for manufacturing the applicator is not particularly limited, and examples thereof include a method in which, first, a roll of paper is cut into a specific size and rolled using a roller to create a shaft. Secondly, cotton fibers that are detangled by a defibration machine are bonded to an end portion of the shaft having an adhesive applied thereto, by appropriately spraying an adhesive to the cotton fibers, thereby wrapping the cotton fibers around the end portion. Thereafter, the aggregate of the wrapped cotton fibers is compression-molded using a mold of a predetermined shape, thereby forming a flat, curved cotton body. After this molding process, the adhesive is dried in a drying step using hot air, a heater, or the like, thereby obtaining the applicator.

The cotton body of the applicator needs to be durable to some extent so that the cotton fibers do not become loose and deformed during use. However, the durability of the cotton body changes significantly depending on the type or amount of the adhesive used for bonding the cotton fibers together. For this reason, when creating the cotton body, it is desirable not only to select the most appropriate adhesive to be used in the step of wrapping the cotton fibers around the shaft, but also to thoroughly bond the cotton fibers together by spraying an appropriate amount of the adhesive thereto to create an aggregate of cotton fibers. For example, the type of the adhesive is preferably polyvinyl alcohol resin and the amount of the adhesive for bonding the cotton fibers is preferably 0.5 mg.

The following examples describe in detail the results of verification tests of the effects of the present invention.

### Examples

Table 1 shows the results of evaluating "rigidity," "amount of cosmetic scooped," and "ease of application" corresponding to the cotton body of the applicator, with the shape of the cotton body being changed variously. The evaluation items such as "user-friendliness (easy of application to fine parts)," "degree of sticking of cosmetic to lips," and "uniform application properties" in relation to the "ease of application" were evaluated by use tests by ten panelists.

The evaluation criteria are categorized as follows: exceptional (pass), excellent (pass), marginal (fail), and poor (fail).

### [Table 1]

**[Table 1]**

| | | | Comparative Example 1 | Comparative Example 2 | Example 1 | Example 2 | Example 3 | Comparative Example 3 | Comparative Example 4 |
|---|---|---|---|---|---|---|---|---|---|
| Shape of Cotton Body | Maximum Width W (unit: mm) | | 3.0 | 4.0 | 4.5 | 5.5 | 6.5 | 7.0 | 8.0 |
| | Thickness T of Thickest Portion (unit: mm) | | 5.0 | 4.0 | 3.6 | 2.8 | 2.0 | 1.5 | 1.0 |
| | Inclination angle θ (unit: °) | | Approximately 30° | | | | | | |
| Evaluation (*1) | Rigidity | | Excellent | Excellent | Excellent | Excellent | Excellent | Marginal | Poor |
| | Amount of Cosmetic Scooped | | Poor | Poor | Exceptional | Exceptional | Exceptional | Marginal | Poor |
| | Ease of Application | User-friendliness (ease of application to fine parts) | Marginal | Excellent | Exceptional | Exceptional | Exceptional | Marginal | Poor |
| | | Degree of sticking of cosmetic to application area (lips) | Poor | Marginal | Exceptional | Exceptional | Exceptional | Marginal | Poor |
| | | Free of unevenness/Uniform application properties | Poor | Marginal | Exceptional | Exceptional | Exceptional | Marginal | Poor |
| | | Overall evaluation | Poor | Marginal | Exceptional | Exceptional | Exceptional | Poor | Poor |

| | | | | | | | | | |
|---|---|---|---|---|---|---|---|---|---|
| *1 "Amount of cosmetic scooped" and "ease of application" were evaluated using lip gloss having viscosity of 56600 mPa·s. | | | | | | | | | |

As shown in Table 1, in Examples 1 to 3 in which the maximum width W of the cotton body was 4.5 to 6.5 mm and the thickness T of the thickest portion was 3.6 to 2.0 mm, the "rigidity" was excellent and "exceptional (pass)" results were obtained for the evaluation items, "amount of cosmetic scooped" and "ease of application."

Table 2 shows the results of evaluating "amount of cosmetic scooped," "durability at one-time use," and "ease of application" when the inclination angle θ of the imaginary line with respect to the shaft was changed, the imaginary line connecting the tip portion of the cotton body and the rear end central portion thereof. The evaluation item, "durability at one-time use," was evaluated by making sure that the inclination angle θ did not change at one-time use.

**[Table 2]**

| | | | Comparative Example 5 | Example 4 | Example 5 | Example 6 | Comparative Example 6 | Comparative Example 7 |
|---|---|---|---|---|---|---|---|---|
| Shape of Cotton Body | Inclination angle θ (unit: °) Maximum Width W: 5.5 mm, Thickness T of Thickest portion: 2.8 mm | | 0° | 25° | 30° | 35° | 45° | 90° |
| Evaluation (*1) | Amount of Cosmetic Scooped | | Poor | Exceptional | Exceptional | Exceptional | Marginal | Poor |
| | Durability at One-time Use (*2) | | Exceptional | Exceptional | Exceptional | Exceptional | Poor | Poor |
| | Ease of Application | User-friendliness (ease of application to fine parts) | Excellent | Exceptional | Exceptional | Exceptional | Poor | Poor |
| | | Degree of sticking of cosmetic to application area (lips) | Marginal | Exceptional | Exceptional | Exceptional | Poor | Poor |
| | | Uniform application properties | Marginal | Exceptional | Exceptional | Exceptional | Poor | Poor |

| | | | | | | | | |
|---|---|---|---|---|---|---|---|---|
| *1 "Amount of cosmetic scooped," "durability at one-time use," and "ease of application" were evaluated using lip gloss having viscosity of 56600 mPa·s. *2 The fact that the inclination angle did not change at one-time use was confirmed. | | | | | | | | |

As shown in Table 2, in Examples 4 to 6 in which the inclination angle θ of the imaginary line with respect to the shaft was 25 to 35 degrees, the imaginary line connecting the tip portion of the cotton body and the rear end central portion thereof, "exceptional (pass)" results were obtained for the evaluation items, "amount of cosmetic scooped," "durability at one-time use," and "ease of application."

Table 3 shows a relationship between each of the shapes of the cotton body and viscosities (degrees of hardness) of viscous cosmetics applied using the cotton body. Using the applicator having the variously shaped cotton body, the "amount of cosmetic scooped" and the "ease of application" were evaluated when the viscous cosmetics of various viscosities (degrees of hardness) were applied.

**[Table 3]**

| | | | Comparative Example 8 | Comparative Example 9 | Example 7 | Example 8 | Example 9 | Comparative Example 10 | Comparative Example 11 |
|---|---|---|---|---|---|---|---|---|---|
| Shape of Cotton Body | Maximum Width W (unit: mm) | | 3.0 | 4.0 | 4.5 | 5.5 | 6.5 | 7.0 | 8.0 |
| | Thickness T of Thickest Portion (unit: mm) | | 5.0 | 4.0 | 3.6 | 2.8 | 2.0 | 1.5 | 1.0 |
| | Inclination angle θ (unit: °) | | Approximately 30° | | | | | | |
| Viscosity of Viscous Cosmetic (mPa·s) | 19300 | Evaluation (Overall evaluation on amount of cosmetic scooped/easiness of application) | Poor/Poor | Marginal/Excellent | Excellent/Exceptional | Exceptional/Exceptional | Excellent/Exceptional | Poor/Marginal | Poor/Marginal |
| | 39500 | | Poor/Poor | Poor/Marginal | Excellent/Exceptional | Exceptional/Exceptional | Excellent/Exceptional | Marginal/Marginal | Poor/Marginal |
| | 56600 | | Poor/Poor | Poor/Marginal | Exceptional/Exceptional | Exceptional/Exceptional | Exceptional/Exceptional | Marginal/Marginal | Poor/Poor |
| Hardness of Viscous Cosmetic | 30 | | Excellent/Poor | Excellent/Poor | Excellent/Exceptional | Exceptional/Exceptional | Excellent/Exceptional | Marginal/Poor | Marginal/Poor |

As shown in Table 3, the use tests were able to confirm that in Examples 7 to 9 in which the maximum width W of the cotton body was 4.5 to 6.5 mm and the thickness T of the thickest portion was 3.6 to 2.0, favorable results were obtained for the "amount of cosmetic scooped" and "ease of application" when viscous cosmetics having a viscosity of 19300 to 56600 mPa·s and a hardness of 30 were applied.

Although disposable, the applicator of the present invention is capable of bringing about the excellent sense of usability especially when applying a viscous cosmetic. Moreover, although data are not provided, the use tests were able to confirm that the same sense of usability as those of applicators that normally accompany cosmetic containers were achieved.

The effects of the type and amount (concentration of an adhesive solution) of the adhesive for bonding the cotton body of the applicator on the durability of the cotton body were evaluated next. There are two types of adhesives for bonding the cotton fibers of the cotton body: a carboxymethyl cellulose solution, and a polyvinyl alcohol solution. The commercially available "UD Swab Shower" (manufactured by Heiwa Medic Inc.) was used as a carboxymethyl cellulose solution product of ordinary (conventional) concentration. The commercially available "UD Swab Universal Shaft" (manufactured by Heiwa Medic Inc.) was used as a polyvinyl alcohol solution product of ordinary (conventional) concentration. For the polyvinyl alcohol solution, a solution having a higher concentration than the ordinary (conventional) concentration used in a commercial product was prepared to create a test cotton body. The test cotton body is usually obtained by spraying an adhesive solution in the step of wrapping the cotton fibers around the shaft and then dried and molded into a predetermined shape, but when a solution having a high concentration of polyvinyl alcohol is used, the cotton body is molded using 0.5-mg polyvinyl alcohol. Note that in the durability test, a plurality of oils having different viscosities were used as test drugs. In the following table, the viscosities of the test drugs indicate the measurements obtained at 25°C.

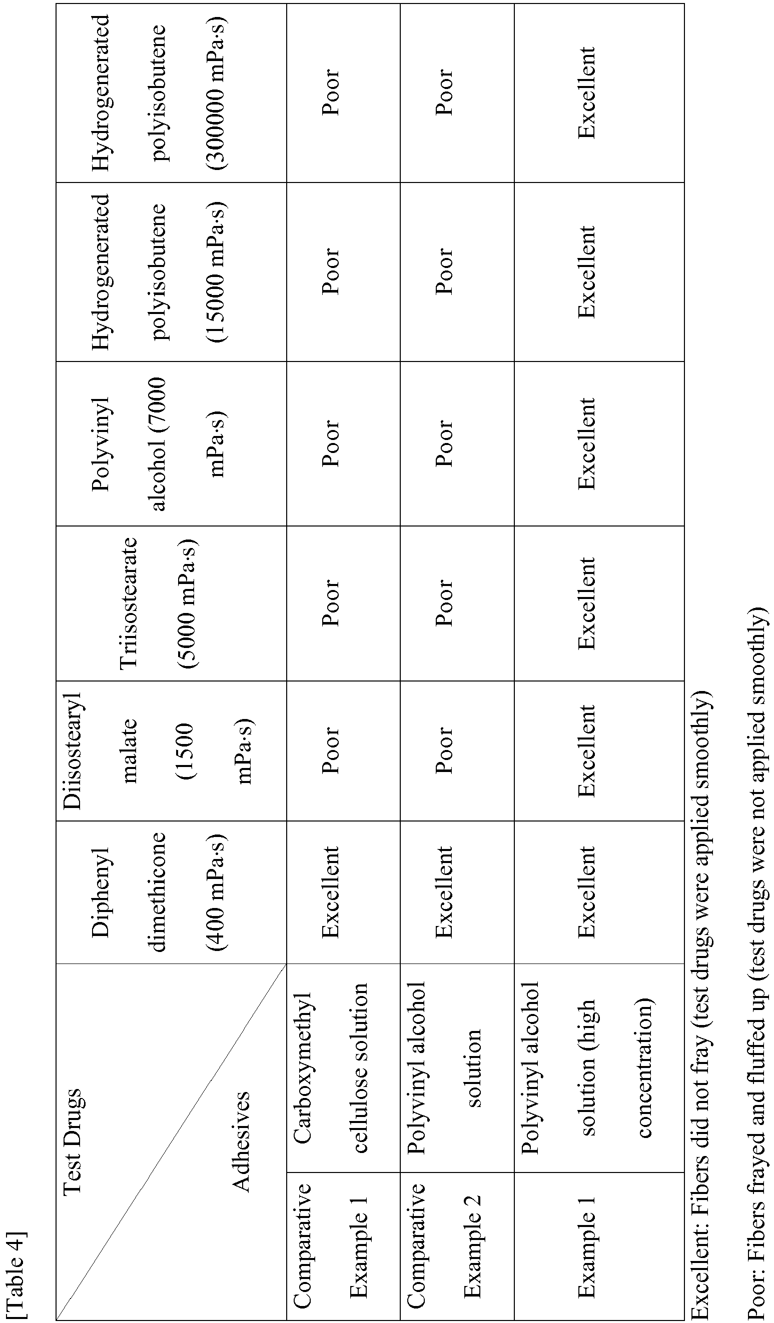

Table 4 shows the results of confirming a fray of fibers when the cotton body of the applicator was immersed in each test drug and then each test drug was applied to a lip after being left for ten minutes. With the polyvinyl alcohol solution (high concentration) of Example 1, the fibers did not fray when any of the test drugs was used, and favorable results were achieved in the viscosity range of 400 to 300000 mPa·s.

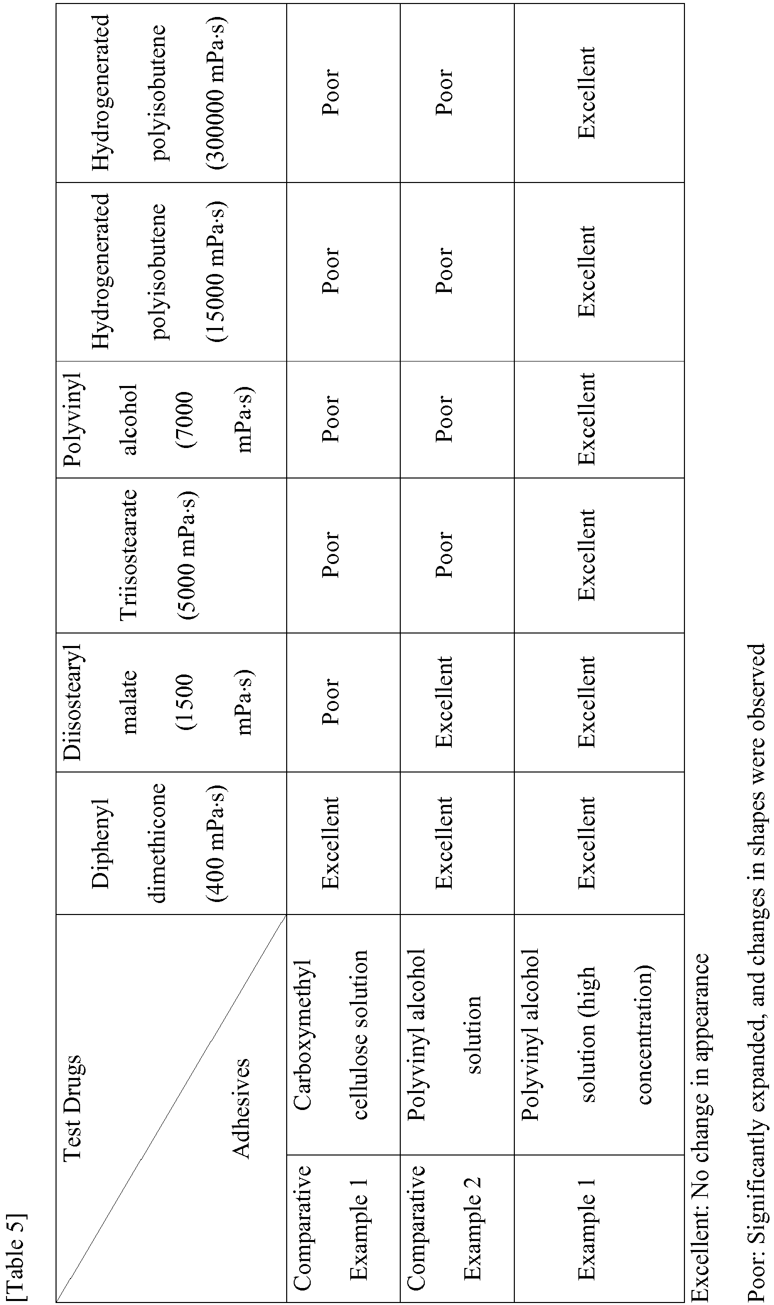

Table 5 shows the results of observing how the fibers frayed after stirring 10 ml of each test drug ten times while having the cotton body of the applicator immersed therein. With the polyvinyl alcohol solution (high concentration) of Example 1, none of the test drugs showed changes in appearance, and favorable results were achieved in the viscosity range of 400 to 300000 mPa·s.

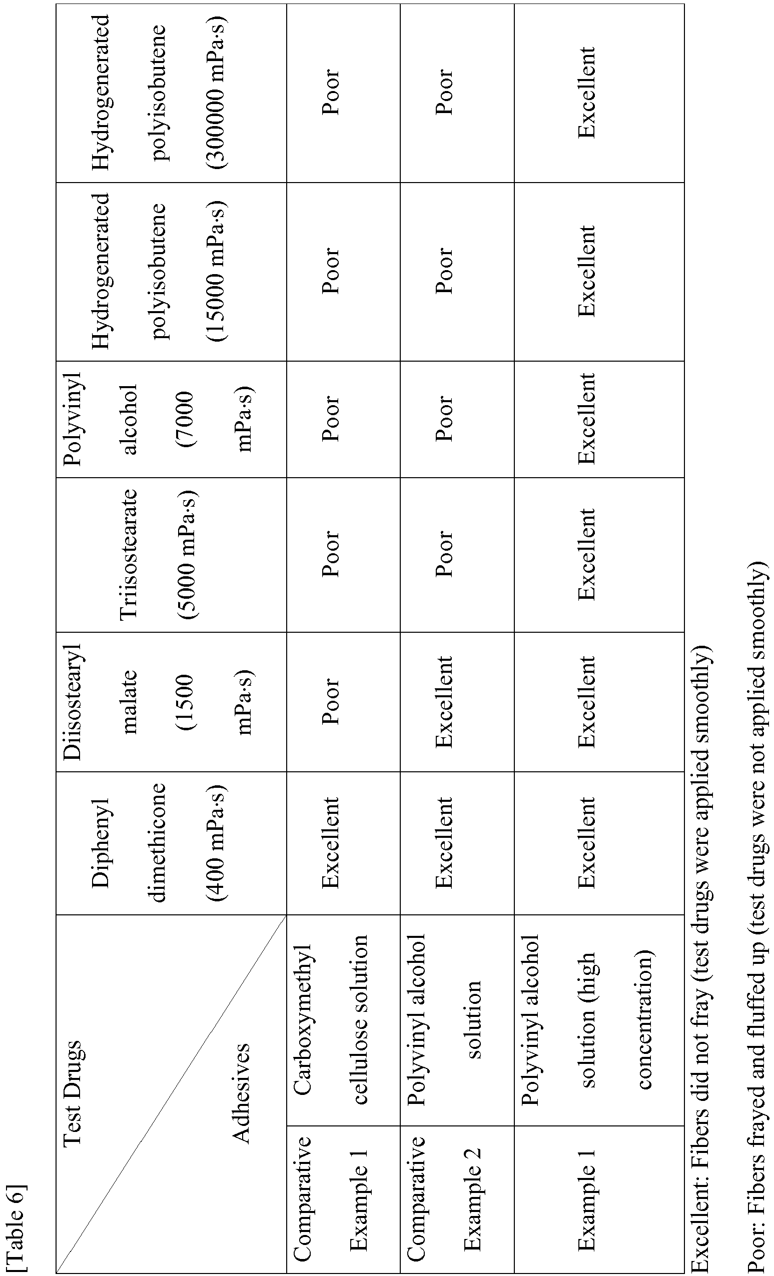

Table 6 shows the results of confirming a fray of fibers when 10 ml of each test drug was stirred ten times while having the cotton body of the applicator immersed therein and thereafter each test drug was applied to a lip. With the polyvinyl alcohol solution (high concentration) of Example 1, the fibers did not fray at any level of viscosity (400 to 300000 mPa·s), and each test drug was applied smoothly.

Fig. 3 shows the results of measuring the hardness of the cotton body. The hardness of the cotton body was measured using the texture analyzer TA XT PLUS (manufactured by Stable Micro Systems) in which, as shown in Fig. 4, a circular disc with a diameter of approximately 1 cm was pressed against the cotton body of the applicator placed on the table and lowered at a speed of 0.1 mm/sec, to measure the stress produced on the cotton body.

As shown in Fig. 3, the results were able to confirm that the cotton body adhered and molded using the polyvinyl alcohol solution (high concentration) had a higher hardness than the cotton body adhered and molded using the carboxymethyl cellulose solution and had strength which can withstand repeated use.

### [Reference Signs List]

- 1: Applicator
- 2: Shaft
- 3: Cotton body
- 4: Imaginary line

## Claims

1. An applicator, comprising a cotton body that extends from an end portion of a shaft and has a flat curved shape in which a maximum width is 4.5 to 6.5 mm and a thickness at a thickest portion is 2.0 to 3.6 mm, wherein an imaginary line connecting a tip portion of the cotton body and a rear end central portion thereof has an inclination angle of 25 to 35 degrees with respect to the shaft.

2. The applicator according to claim 1, wherein the cotton body is formed of an aggregate of cotton fibers bonded together with 0.5 mg of polyvinyl alcohol resin.

3. A viscous cosmetic applicator, comprising a cotton body that extends from an end portion of a shaft and has a flat curved shape in which a maximum width is 4.5 to 6.5 mm and a thickness at a thickest portion is 2.0 to 3.6 mm, wherein an imaginary line connecting a tip portion of the cotton body and a rear end central portion thereof has an inclination angle of 25 to 35 degrees with respect to the shaft.

4. The viscous cosmetic applicator according to claim 3, wherein the cotton body is formed of an aggregate of cotton fibers bonded together with 0.5 mg of polyvinyl alcohol resin.

5. The viscous cosmetic applicator according to claim 4, which is used for applying a viscous cosmetic having a viscosity of 400 to 300000 mPa·s.

6. A method for using an applicator, the method comprising scooping and applying a viscous cosmetic or a viscous pharmaceutical preparation by using the applicator according to claim 1 or 2.

7. A method for using a viscous cosmetic applicator, the method comprising scooping and applying a viscous cosmetic by using the viscous cosmetic applicator according to any one of claims 3 to 5.
